# EUROPEAN PATENT SPECIFICATION

(11) **EP 0 681 819 B1**
(45) Date of publication and mention of the grant of the patent: **30.12.1998**
(21) Application number: 95107182.8
(22) Date of filing: 11.05.1995
(51) Int. Cl.: A61F 13/15

(54) **Absorbent articles having raised portions**
Absorbierender Artikel mit erhöhten Teilen
Article absorbant comprenant des parties élevées

(30) Priority: 12.05.1994 US 241826
(43) Date of publication of application: 15.11.1995
(73) Proprietor: McNEIL-PPC, INC., Milltown New Jersey 08850 (US)
(72) Inventor: Stevenson, Philip J., Princeton, NJ 08540 (US); Holtman, Dennis C., Flemington, NJ 08822 (US)
(74) Representative: Groening, Hans Wilhelm, Dipl.-Ing.

(56) References cited:
- EP-A- 0 405 403
- US-A- 2 834 347
- US-A- 4 631 062
- US-A- 4 883 481
- US-A- 5 127 911

## Description

This invention relates to absorbent articles that are worn against the body for absorbing body fluids, and in particular, to shapable articles such as sanitary napkins and diapers in accordance with the preamble of claim 1. An article of this type is known from EP-A-0 405 403.

### Background of the Invention

US-A-5,127,911 discloses a sanitary feminine napkin having an oblong shape and containing a fluid absorbent core interposed between a fluid impermeable backing and a fluid permeable cover. Said napkin comprises a mechanism which allows a user the option of forming a contoured projection in said napkin. Said mechanism comprising a string means, which, when pulled, draws together part of the longitudinal edges of said napkin and forms said contoured projection.

EP-A-0 405 403 relates to a napkin as a liner for a panty having a fluid-impermeable layer, an absorbent body and a fluid-permeable cover. The napkin has at least one longitudinal fold which allows an adaptation of the width of the liner to the width of the crotch portion of a panty. Said fold is releasably and at least partially closed by means of adhesive in the longitudinal fold arranged to join the two sides of the longitudinal fold to one another. The adhesive can also be arranged transversely to the longitudinal fold. Additionally, the napkin can have lateral wings at both longitudinal edges of the napkin for additional fixing of the wings to the outside of the crotch portion of a panty.

Numerous forms of absorbent articles have been proposed that are specially shaped to adapt to the region of the body from which they absorb fluids. Rectangular diapers and oval catamenial pads have been replaced for the most part by specially shaped articles, such as diapers with wrap around portions and hourglass-shaped pads.

In addition to developments in the geometry of absorbent articles, it has been realized that the body is a three dimensional surface, and some designs have proposed accommodating this fact. For example, U.S. Patent Nos. 4,631,062 and 4,804,380 both to Lassen et al., disclose sanitary napkins that are mechanically shaped to have an upward tuck at an end portion. A similar upward extending portion is shown in U.S. Patent No. 3,092,109 -- Mosier which is formed by stitching a portion of the pad into a fold. An interlabial pad is formed by folding an oval pad and affixing the oval structure to a backing sheet. Additionally, it is known to change the cross-section of an absorbent structure by securing portions of the surface into a fold with an adhesive strip or tab applied to the surface of the article, as disclosed in U.S. Patent No. 2,834,347 -- Connolly and U.S. Patent No. 4,883,481 -- Blanchard et al.

There remains, however, a need to provide three dimensional features, i.e., changes in profile or cross-section of absorbent articles, efficiently and by using technology that is readily incorporated into existing products. Moreover, such improvements must be of a nature that will be readily accepted by the consumer. Therefore, it would be desirable to form such changes in profile by folding flat portions of an absorbent pad, since flat absorbent articles are easily produced and more readily shipped and stored. Therefore, it is an object of the present invention to provide improved absorbent articles having raised portions that are formed from conventional flat absorbent pads. A further object of the present invention is to provide methods for making such improved absorbent articles that take advantage of the equipment and production techniques currently found in the industry, thereby rendering the designs disclosed efficient and economical to manufacture. Finally, it is another object of the present invention to provide absorbent articles that may be shaped by the consumer, immediately prior to their application.

### Summary of the Invention

These and other objects are met by the provision of an absorbent article having the features of the characterizing portion of claim 1. By folding the absorbent article to adhere portions of the garment facing side together using the adhesive, raised portion is created. In certain embodiments the adhesive is disposed along the longitudinal axis of the absorbent article, while in others, the adhesive as well as additional areas of adhesive are disposed adjacent the longitudinal axis. Thus, in some embodiments the article is folded to place the adhesive area in contact with itself, while in others the article is folded to place the adhesive area in contact with the garment facing side. Preferably, the absorbent articles of the present invention are in the form of catamenial pads or diapers.

### Brief Description of the Drawings

FIG. 1 is a plan view of an oval pad made in accordance with the present invention, before folding.
FIG. 2 is an end view of the pad shown in FIG. 1.
FIG. 3 is a side view of the pad shown in FIGS. 1-2 that has been folded in accordance with the present invention to create a raised portion.
FIG. 4 is an end view of the pad shown in FIG.3.
FIG. 5 is a perspective view of the pad depicted in FIGS. 3-4.
FIG. 6 is a plan view of another embodiment of an oval pad made in accordance with the present invention, before folding.
FIG. 7 is a side view of the pad shown in FIG. 1 that has been shaped by folding in accordance with the present invention to create a raised portion.

### Detailed Description of the Preferred Embodiments

The present invention provides absorbent articles such as catamenial pads and diapers that have one or more areas of adhesive, preferably in the form of longitudinal strips, disposed on the garment facing side of the absorbent article. By folding the garment facing side toward itself, the adhesive area adheres to either itself or to the garment facing side, thereby creating a raised portion that, in the example of a catamenial pad, would conform to certain portions of the perineal region of the user.

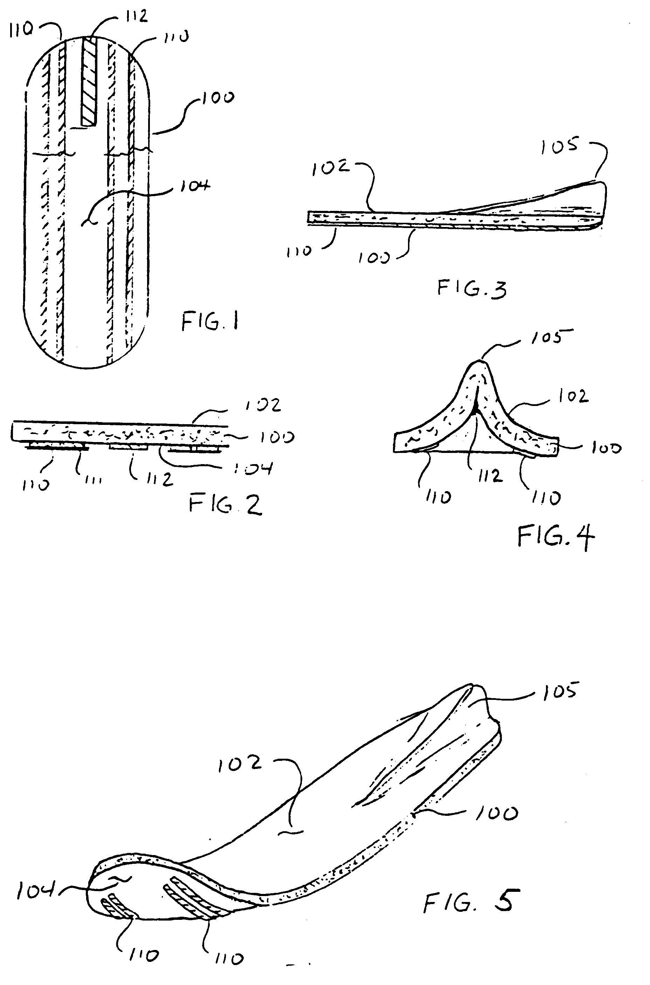

Referring now to FIG. 1, there is shown a top view of an absorbent article 100 made in accordance with the present invention. The article 100 illustrated in FIG. 1 is an oval catamenial pad. However, it will be understood that the invention is in no way limited to the particular embodiments that are described with reference to this and the other drawing figures. Generally, as shown in Fig. 2, the absorbent article 100 has a body facing side 102 and a garment facing side 104. As will be understood by those of ordinary skill, the absorbent article 100 may comprise an absorbent batt made of, for example, cellulosic fibers. In certain embodiments, however, the absorbent article will comprise a multiple layer structure that may include fluid absorbing layers, fluid distribution layers, or facing layers on either the body facing side 102, the garment facing side 104, or both. Thus, for purposes of the present invention, the term "absorbent article" is to be construed broadly to encompass any construction of such articles.

An end view of the absorbent article 100 of FIG.1 is shown in FIG. 2. As explained above, the absorbent articles typically found in the prior art and which are useful with the present invention are substantially flat. However, those of ordinary skill will appreciate that the invention disclosed herein is readily adapted and used with structures that are curved, hemispherical, or which have a convoluted surface or other pattern on either the body facing side 102 or the garment facing side 104. As seen in FIG. 2, a plurality of adhesive strips 110, 112 are arranged on the garment facing side 104 of the absorbent article 100. The formulation of these strips and their application is well known and is readily implemented using currently available production equipment. As seen in FIG. 2, release paper 111 preferably covers the adhesive strips prior to the application of the absorbent article 100 by the user, particularly when it is in the form of a catamenial pad, wherein certain of the adhesive strips 110 are used to adhere the garment facing side 104 to an undergarment.

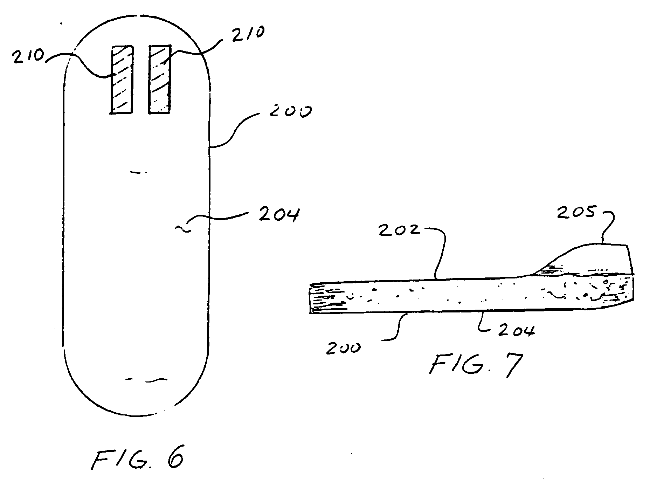

Referring now to FIGS. 3-5, an absorbent article 100 made in accordance with the present invention and ready for use is shown. As seen in the side view of FIG. 3 and the end view of FIG. 4, in a preferred embodiment, the centrally disposed adhesive strip 112 shown in FIGS> 1-2 is folded upon itself to create a raised portion 105 in the body facing side 102 of the absorbent article 100. In the embodiment illustrated, the center strip 112 is located along a portion of the longitudinal axis of the absorbent article 100 and does not run the full longitudinal length. Thus, as seen in FIGS. 3-5, the raised portion 105 is located at the end of the absorbent article where the adhesive is located, but tapers off toward the center. These features can also be seen in the perspective view of FIG. 5, which is oriented with the body facing side 102 up, and in which a portion is upturned to show the adhesive strips 110 that are used to attach the absorbent structure 100 to a garment.

Another embodiment of an absorbent article 200 made in accordance with the present invention is show in FIGS. 6-7. In this embodiment, the absorbent article 200 is made more convenient by using two strips of adhesive 210 for shape formation. Release paper is not needed if an adhesive is chosen that will not adhere to the garment facing side 204, but will instead only adhere to itself. When the embodiment illustrated in FIG. 6 is folded, the profile is as shown in FIG. 7. As shown, a raised portion of a more sharply defined profile is formed.

As illustrated by the two embodiments described above, the length and position of the adhesive 112,210 that forms the raised portion 105,205 can vary depending upon the final product shaped desired. If the raised portion 105, 205 is in the rear of a catamenial pad, as illustrated, then the pad fits closely to the body between the buttocks thus preventing leakage in the lying down position. However, it will be readily appreciated by those of ordinary skill that other types of designs will solve other fluid control and comfort problems. For example, more than one raised portion could be created, or raised portions running transversely, or at angles to the longitudinal axis of the absorbent structure can be constructed. In any embodiment, however, a major advantage of the present invention is that the absorbent article is made substantially flat in production and remains flat until used.

Thus, a method of forming shaped absorbent articles that have at least one strip of adhesive applied to their garment facing side is provided in that a first portion of the body facing side is folded toward a second portion of the body facing side. Since the first and second portions are disposed substantially adjacent the adhesive, they are adhered together by the adhesive. In some embodiments, the step of adhering will require folding the adhesive on to itself, while in other embodiments it will be folded on to another strip of adhesive, or the garment facing side.

Although certain embodiments of the present invention have been described herein with particularity, these descriptions have been for the purpose of illustrating the invention and are not meant to be limiting. As explained above, numerous variations as to the shape, construction and use of the absorbent products described herein are contemplated and will be immediately apparent to those of ordinary skill upon review of the foregoing specification. Accordingly, reference should be made to the appended claims in order to determine the true scope of the present invention.

## Claims

1. An absorbent article (100, 200) having a body facing side (102, 202) and a garment facing side (104, 204), and at least one area of adhesive (110, 112, 210) disposed on the garment facing side (104, 204) such that the absorbent article (100, 200) is foldable to adhere portions of the garment facing side (104, 204) together thereby creating a raised portion (105, 205) protruding above the body facing side (102, 202) of the article (100, 200), characterized in that the at least one area of adhesive (110, 112, 210) is disposed at the rear end of the absorbent article (100, 200) in order to provide the raised portion (105, 205) at the rear end of the absorbent article (100, 200) tapering off towards the center portion thereof and fitting closely to a wearer's body between the buttocks of the wearer.

2. The absorbent article (100) of claim 1, having a longitudinal axis, wherein at least one area of adhesive (112) is disposed along the longitudinal axis.

3. The absorbent article (100, 200) of claim 1 or 2, comprising at least one area of adhesive (110, 210) disposed adjacent the longitudinal axis.

4. The absorbent article (100) of claim 1, wherein the article (100) is foldable to place the adhesive area (112) in contact with itself.

5. The absorbent article (100, 200) of claim 1, wherein the article (100, 200) is foldable to place one adhesive area (110, 210) in contact with another adhesive area (110, 210).

6. The absorbent article (100, 200) of claim 1, wherein the article (100, 200) is foldable to place the adhesive area (110, 112, 210) in contact with the garment facing side (104, 204).

7. The absorbent article (100, 200) of claim 1 in the form of a catamenial pad.

8. The absorbent article (100, 200) of claim 1, in the form of a diaper.

## Patentansprüche

1. Absorbierender Artikel (100, 200) mit einer zum Körper weisenden Seite (102, 202) und einer zur Kleidung weisenden Seite (104, 204), und zumindest einer Klebstoffzone (110, 112, 210), die auf der zur Kleidung weisenden Seite (104, 204) angeordnet ist, so daß der absorbierende Artikel (100, 200) faltbar ist, damit Bereiche der zur Kleidung weisenden Seite (104, 204) aneinander haften, wodurch ein erhöhter Bereich (105, 205) geschaffen wird, der über die zum Körper weisende Seite (102, 202) des Artikels (100, 200) vorsteht, dadurch gekennzeichnet, daß die zumindest eine Klebstoffzone (110, 112, 210) am hinteren Ende des absorbierenden Artikels (100, 200) angeordnet ist, um den erhöhten Abschnitt (105, 205) am hinteren Ende des absorbierenden Artikels (100, 200) vorzusehen, der sich zu dessen mittlerem Abschnitt hin verjüngt und eng am Körper einer Trageperson zwischen den Gesäßbacken der Trageperson anliegt.

2. Absorbierender Artikel (100) nach Anspruch 1, mit einer Längsachse, wobei zumindest eine Klebstoffzone (112) entlang der Längsachse angeordnet ist.

3. Absorbierender Artikel (100, 200) nach Anspruch 1 oder 2, umfassend mindestens eine Klebstoffzone (110, 210), die der Längsachse benachbart angeordnet ist.

4. Absorbierender Artikel (100) nach Anspruch 1, wobei der Artikel (100) faltbar ist, um die Klebstoffzone (212) in Kontakt mit sich selbst zu bringen.

5. Absorbierender Artikel (100, 200) nach Anspruch 1, wobei der Artikel (100, 200) faltbar ist, um eine Klebstoffzone (110, 210) mit einer anderen Klebstoffzone (110, 210) in Kontakt zu bringen.

6. Absorbierender Artikel (100, 200) nach Anspruch 1, dadurch gekennzeichnet, daß der Artikel (100, 200) faltbar ist, um die Klebstoffzone (110, 112, 210) mit der zur Kleidung weisenden Seite (104, 204) in Kontakt zu bringen.

7. Absorbierender Artikel (100, 200) nach Anspruch 1 in Form einer Monatsbinde.

8. Absorbierender Gegenstand (100, 200) nach Anspruch 1 in Form einer Windel.

## Revendications

1. Article absorbant (100, 200) ayant un côté faisant face au corps (102, 202) et un côté faisant face au vêtement (104, 204) et au moins une surface d'adhésif (110, 112, 210) disposée sur le côté faisant face au vêtement (104, 204) de sorte que l'article absorbant (100, 200) est pliable pour faire adhérer les portions du côté faisant face au vêtement (104, 204) de façon à produire une portion élevée (105, 205) en saillie au dessus du côté faisant face au corps (102, 202) de l'article (100, 200), caractérisé en ce qu'au moins une surface de l'adhésif (110, 112, 210) est disposée à l'extrémité arrière de l'article absorbant (100, 200) afin de fournir la portion élevée (105, 205) à l'extrémité arrière de l'article absorbant (100, 200) en effilement vers la portion centrale et en adaptation étroite au corps de l'utilisateur entre les fesses de l'utilisateur.

2. Article absorbant (100) selon la revendication 1, ayant un axe longitudinal, dans lequel au moins une zone d'adhésif (112) est disposée le long de l'axe longitudinal.

3. Article absorbant (100, 200) selon la revendication 1 ou 2, comprenant au moins une zone d'adhésif (110, 210) disposée de façon adjacente à l'axe longitudinal.

4. Article absorbant (100) selon la revendication 1, dans lequel l'article (100) est pliable pour placer la surface adhésive 112) en contact avec elle-même.

5. Article absorbant (100, 200) selon la revendication 1, dans lequel l'article (100, 200) est pliable pour placer une surface adhésive (110, 210) en contact avec une autre surface adhésive (110, 210).

6. Article absorbant (100, 200) selon la revendication 1, dans lequel l'article (100, 200) est pliable pour placer la surface adhésive (110, 112, 210) en contact avec le côté faisant face au vêtement (104, 204).

7. Article absorbant (100, 200) selon la revendication 1, sous forme d'un tampon cataménial.

8. Article absorbant (100, 200) selon la revendication 1, sous forme d'une couche.
